# EUROPEAN PATENT SPECIFICATION

(11) **EP 4 568 736 B1**
(45) Date of publication and mention of the grant of the patent: **30.09.2026**
(21) Application number: 23768613.4
(22) Date of filing: 10.08.2023
(51) Int. Cl.: A61N 1/04, A61B 5/256, A61B 5/268, A61B 5/282, A61B 5/00, A41D 1/00, A41D 13/12

(54) **SENSORISED WEARABLE TEXTILE DEVICE, GARMENT INCORPORATING THE SAME AND CORRESPONDING MANUFACTURING METHOD**
SENSORISIERTE TRAGBARE TEXTILVORRICHTUNG, KLEIDUNGSSTÜCK DAMIT UND ZUGEHÖRIGES HERSTELLUNGSVERFAHREN
DISPOSITIF TEXTILE PORTABLE MUNI DE CAPTEURS, VÊTEMENT L'INCORPORANT ET PROCÉDÉ DE FABRICATION CORRESPONDANT

(30) Priority: 10.08.2022 IT 202200017160
(43) Date of publication of application: 18.06.2025
(73) Proprietor: Accyourate Group SpA, 67100 L'Aquila (IT)
(72) Inventor: ARNALDO USAI, Giuseppe, 67100 L'Aquila (IT); GIULIANI, Andrea, 67100 L'Aquila (IT); AGRUSTI, Andrea, 67100 L'Aquila (IT)
(74) Representative: Mola, Edoardo
(86) International application number: PCT/IB2023/058118
(87) International publication number: WO 2024/033881

(56) References cited:
- GB-A- 2 594 335
- GB-A- 2 596 047
- US-A1- 2013 041 272
- US-A1- 2013 123 601
- US-A1- 2019 261 921

## Description

### TECHNICAL FIELD

The present invention refers to a sensorized wearable textile device for detecting electrical signals for estimating the electrocardiogram and respiratory rate of a user wearing this device. The latter can be provided in a garment for greater precision in the position of the sensors and for greater ease of handling even by untrained users, e.g. staff of service companies, industries, etc. who wear the device while working.

### STATE OF ART

Garments are known for detecting electrical signals feeding known algorithms for estimating the electrocardiogram and respiratory rate. In sports, the detection of electrical signals is favored by sweat and implementation is simplified since training sessions have a relatively limited duration.

However, when it is necessary to collect worker data during working hours, it is important to identify a manufacturing layout suitable for large number production, precise enough and comfortable to wear for long periods.

GB-A1-2596047 discloses a multiplayer structure for measuring hearth rate and, thus, functioning also when the sensing electrodes are wet e.g. by sweat and pressed on the skin with a relatively low pressure.

### SCOPES AND SUMMARY OF THE INVENTION

The scope of the present invention is to provide a sensorized textile device capable of satisfying at least in part the needs indicated above.

The scope of the present invention is achieved by means of an elastic band textile device for adherence to the body as defined by claim 1.

The present invention is defined by the appended claims.

A sensorized band configured in this way provides a sufficient comfort when worn for long periods through the first textile layer, being in particular of textile material the area around the electrodes.

The multilayer construction is simple to manufacture and to provide large production batches and is flexible enough around the chest to have adequate comfort over long periods of use and adapts to the user's chest.

In the same way, the electrode unit obtained from a subsequently cut screen-printed sheet allows to reduce waste to a minimum, to precisely control the deposition of the ink and have precise geometries of the component and prepare the process for large production batches. Furthermore, through screen printing, it is possible to incorporate the pad and the elongated filiform conductor that carries the electrical signal to the control unit in a single element, simplifying the production process.

The use of adhesive on both layers sheathes the filiform conductor in order to make adhesion efficient and at the same time reduce the disturbances of the electrical signal along the filiform conductor.

Other advantages of the present invention are discussed in the description and referred to in the dependent claims.

### BRIEF DESCRIPTION OF THE DRAWINGS

The invention is described below on the basis of non-limiting examples illustrated by way of example in the following figures, which refer respectively to:
- Fig. 1 a plan view of some electrode assemblies for a device according to the present invention;
- Fig. 2 an enlarged schematic view of a section of the device in a manufacturing phase prior to gluing;
- Fig. 3 an enlarged schematic view of a section of the device on the skin side; and
- Fig. 4 an enlarged schematic view of a section of the device on the opposite skin side; and
- Fig. 5 an enlarged perspective view of an electrode unit and a mechanical connection element for electric conduction applied thereon.

### DETAILED DESCRIPTION OF THE INVENTION

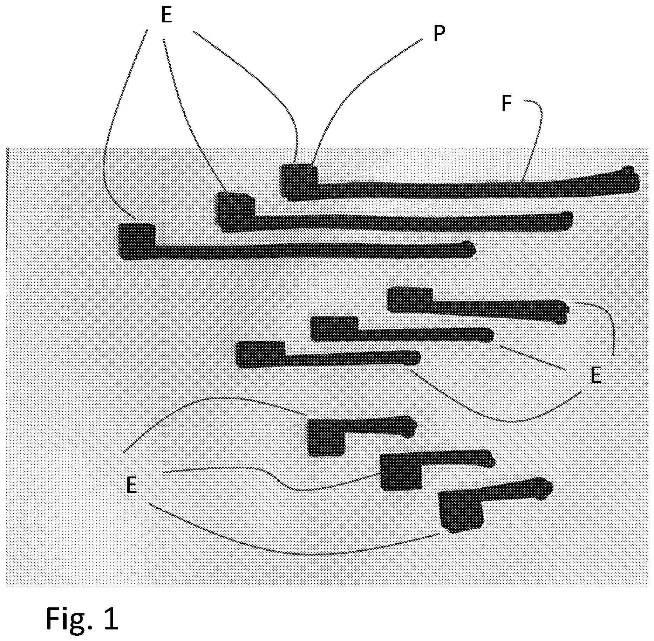

Figure 1 shows electrode units E for a wearable textile device according to the present invention.

These electrode units E are preferably made starting from a sheet screen-printed almost completely with an electrically conductive ink, e.g. a peripheral portion of the sheet is not screen printed.

On this screen-printed area several electrode units E are obtained by cutting the sheet, for example by laser cutting. Preferably, as illustrated in Figure **1****,** the cutting operation includes changes in direction and/or non-straight cutting lines to obtain the shaped peripheral edge of each electrode assembly. Therefore, the conductive ink is present on the entire cut area or in any case on most of it and the conductive ink is found on the entire cut perimeter or on most of it. In this way, it is possible to obtain geometries of electrode units E to adapt to the three-dimensional configuration of the band. For example, the configuration of electrode units E illustrated in the figures is substantially one-dimensional so as to affect the bending and torsional stiffness of the band as little as possible, thus increasing comfort.

Screen printing provides a greater control of the uniformity of the thickness of the conductive ink compared to other techniques such as spraying, and is aimed at maximum reduction of the thickness of the "added" materials on the sheet, in order to cancel the perception of "foreign bodies" on the sensorised and skin-contact portions of tissue. Additional techniques for applying electrically conductive ink include immersion in a bath or the use of screen printing masks through which the ink is sprayed. The application of the ink on the sheet is such that the thickness of the ink is substantially uniform, in particular the ink has uniform thickness on the electrode units E.

According to a preferred embodiment, the sheet is made of fabric, so as to increase the comfort of direct contact with the skin. Even more preferably, the fabric comprises a yarn based on polyester (PE) with additives of ceramic and elastane (EA) particles. For example, ceramic particles are oxides of one or more metals, such as zinc, platinum, titanium, aluminium and silicon. The percentages of these particles do not exceed 5% by weight and the additives can be either by incorporation into the polyester before extrusion of the yarn or by coating on the yarn. The use of these particles makes it possible to increase the electrical insulation of the elongated filiform conductor and reduce the disturbances along this conductor on the electrical signal picked up by electrode unit E, in particular in the presence of the user's sweat. However, it should be noted that sweat increases and stabilizes the conductivity of the electrically conductive polymer in contact with the skin.

According to a preferred embodiment, the conductive polymer comprises polyethylenedioxythiophene:sulphonated polystyrene, hereinafter PEDOT:PSS, and at least one of dimethylsulfoxide DMSO, ethylene glycol EG and sorbitol, preferably the former in a greater quantity by weight than the latter. Alternatively or in combination, it is also possible to functionalize the polymer through metallic nanoparticles, for example Ag, Au, Ag/AgCl. It is also possible to add a crosslinker to the polymer, for example (3-glycidyloxypropyl)trimethoxysilane (GLYMO) to improve the mechanical characteristics, and/or a surfactant, for example Triton X or p-dodecylbenzenesulfonic acid (DBSA), to improve application on the sheet by screen printing.

As illustrated in figure **1****,** each electrode unit E comprises, preferably in a single body cut from the sheet, a pad P in use in contact with the user's skin and an elongated filiform element F coming out of pad P. As illustrated in the drawings, elongated filiform element F is narrower than pad P and carries the electrical signal detected by pad P, which acts as an electrode, through the screen-printed layer of electrically conductive ink which covers the entire functional surface of electrode unit E. Preferably, ink layer on pad P is not in relief with respect to that on the elongated filiform element F.

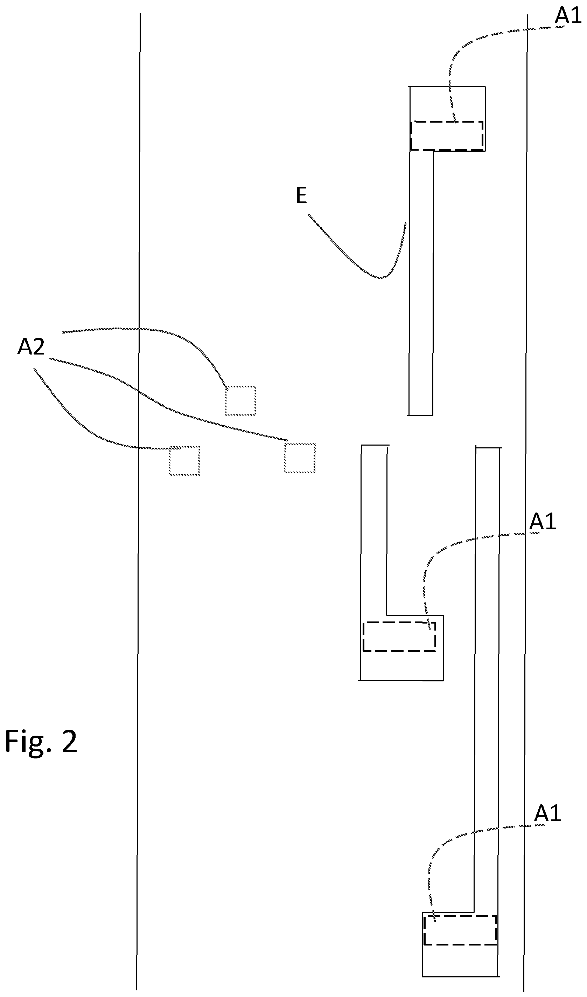

Figure 2 schematically shows a further production step of the textile device of the present invention wherein electrode units E, e.g. at least three for the detection of signals suitable for estimating the user's electrocardiogram, are coupled, on the opposite side of the screen printed surface, with an adhesive e.g. a hot-laminate adhesive film such as a thermoplastic polyurethane (TPU) based adhesive. For example, the film has 40% elongation with a 4N load and 100% elastic recover when applied to a 10mm tape. In order to facilitate the production method, the adhesive is a double-sided adhesive film.

Furthermore, the textile device of the present invention comprises a first textile layer S1 in use in contact with the skin and a second layer S2, preferably also textile and even more preferably of the same fabric as textile layer S1, in particular in a single body with layer S1 as illustrated in figure 2. Preferably, textile layer S1 is made with 50% Polyurethane fibers (Pu), 36% Polyamide fibers (Pa) and 14% Elastane fibers (Ea).

With reference to figure 2, a further layer of adhesive is applied to a band of fabric, preferably the same as electrode unit E.

Furthermore, in order to allow the electrical signals to be picked up and transmitted, first layer S1 has openings A1 through which pad P is used in contact with the skin. Pad P is superimposed on first layer S1 towards the skin and the remaining part of the electrode unit E, through the corresponding opening A1, is separated from the skin through the first layer S1. In particular, pad P, as shown in figure 2, is on first layer S1 out of the corresponding opening A1 along a direction of elongation of elongated filiform conductor F. This ensures good contact of pad P with the skin. As shown in figure 2, openings A1 are slots having at least a maximum dimension smaller than a maximum dimension of corresponding pad P. In this way, waste material is reduced. For example, a larger side of opening A1 is smaller than a larger side of pad P.

Similarly, second openings A2 are made on the second layer S2 to expose the surface of an electrical terminal T electrically connected to the elongated filiform conductor F. Preferably, the electrical terminal T is made of an electrically conductive metal and is arranged in relief with respect to the second layer S2. For example, the electrical terminal T is crimped to the filiform electrical conductor F.

According to a preferred embodiment of the present invention, the textile device is further provided with a boss B of a thermally conductive material e.g. metallic having a first surface arranged in use in contact with the skin and a second surface uncovered on the second layer, such that the second surface is representative, after a suitable heat transmission time, of the temperature of the skin.

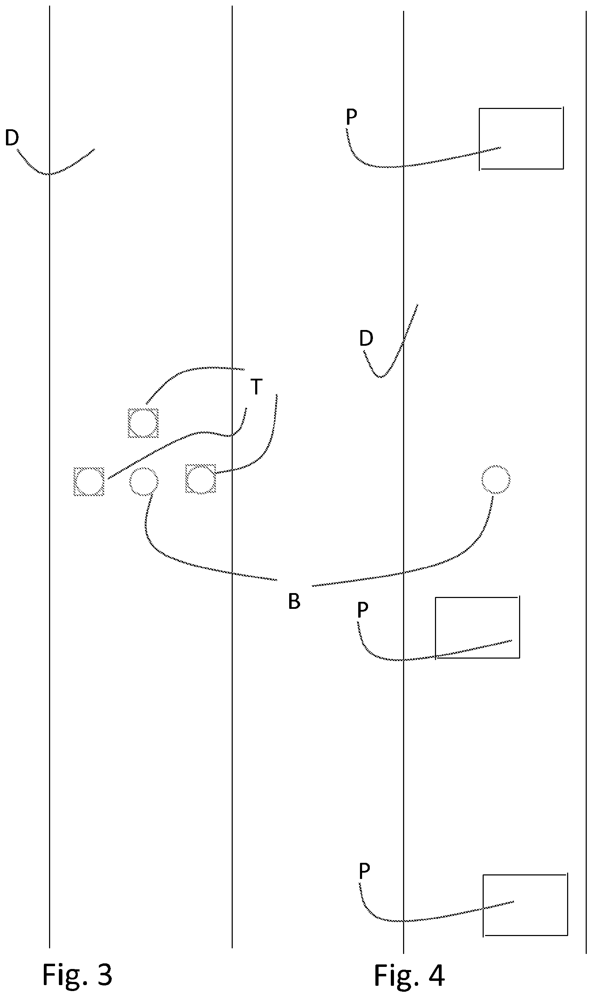

Preferably, terminals T and boss B are close together so that an electronic control unit, releasably carried on the textile device, is simultaneously connected to exchange electrical and temperature signals with terminals T and boss B. For example, at least one between terminals T and boss B acts both as a signal transmission element and as a mechanical attaching element for the electronic control unit. For example, boss B is a male or female element of a snap button that releasably connects the control unit to band D. Furthermore, as shown in figure 3, terminals T and boss B are arranged in a non-axisymmetric figure so that the control unit has only one fixing angular position on band D.

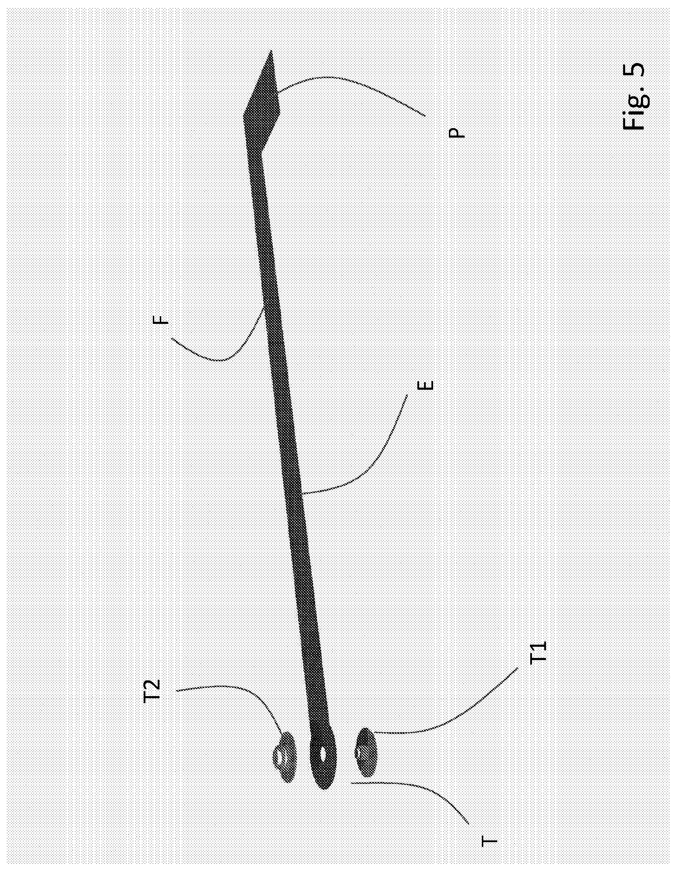

According to an embodiment, at least one of the terminals T is also a male or female element of a snap button. Furthermore, as illustrated in figures 3 and 4, terminals T, unlike boss B, are separated from the skin thanks to first textile layer S1 so as not to touch the latter. Furthermore, terminals T are also protected by the adhesive film since the latter is arranged between terminals T and first textile layer S1. Figure 5 shows an electrode unit E to which terminal T is connected e.g. by crimping, having a first and a second flange element T1, T2 rigidly connected one inside the other, first element T1 being of an electrically conductive material in contact with the electrically conductive ink and, therefore, in electrical continuity with pad P. The first and second flange elements T1, T2, when rigidly connected to each other, compress a corresponding portion of electrode unit E.

To complete the textile device, first layer S1 is glued onto second layer S2, in the embodiment of figure 2, the layers are folded lengthwise over each other to define a band D wearable around the chest (figures 3 and 4). In this way, moreover, the adhesive sheaths the elongated filiform conductor F and further shields the electrical signal carried from disturbances, for example present due to the user's sweat. Therefore, an adhesive material, preferably a bi-adhesive film, more preferably the same bi-adhesive film described above is placed on both faces of elongated filiform conductor F. Preferably, the bonding of first layer S1, second layer S2 and electrode units E is achieved by hot pressing. Furthermore, in this configuration with a band height of 4 cm, applying a load of about 20 N results in 30% elongation and full elastic recover i.e. by 100%. It must be considered that, given the use of cardiac and respiratory monitoring for many hours, uses by children are also foreseen and, consequently, the height is purely indicative and must be suitable for the user.

A suitable closure device (not shown) adjusts the mutual position of corresponding portions of the ends of the band B so that the user can adapt the band itself to his own chest. The closure device can be made in various ways, both 'open/closed' and with continuous adjustment, for example through a zipper in which each side is fixed to the relative end portion: press-studs or traditional buttonholes applied to the end portions, a buttonhole carried by one end portion and cooperating with the other end portion in a sliding manner to be subsequently fixed by means of hook and loop, laces passing between the end portions and fixed either by means of a knot or by means of a sliding element with or without a spring commonly used to adjust in the circumferential direction anoraks and/or the tension of sports shoe laces etc. In general, the band is such that the first and second end portions face each other in the circumferential direction and approach each other when the closure device is tightened.

In particular, the closure device is manually adjustable by the user between a release configuration in which the band is not stretched and the wearable element is worn easily, and a tightening configuration in which band D is stretched in the circumferential direction to apply desired and sufficient pressure to the skin.

According to an alternative embodiment not illustrated band D is connected e.g. by stitching to a garment such as a top with straps, sleeveless shoulders or with sleeves in order to make it even more comfortable to wear for many hours e.g. the entire working day, as well as keeping band D firmly in place.

Finally, it is clear that modifications or variations can be applied to the textile device described and illustrated here without thereby departing from the scope of protection as defined by the attached claims.

For example, according to an embodiment not shown, terminals T and boss B are magnetic and preferably substantially flat. Consequently, the electronic control unit also comprises magnets e.g. permanent magnets, so the mechanical support of the electronic control unit on band B and the electrical data exchange connection between electrode units E and the electronic control unit are performed via a single magnetic device.

## Claims

1. Band textile device (D) comprising:
at least one electrode unit (E) made by applying an electrically conductive polymer on a flexible sheet
the electrode unit comprising a pad (P) in contact with the skin of a user in use and an elongated filiform element (F) defining an electrical continuity with the pad
the electrode unit being cut from sheet on which the electrically conductive polymer is previously applied
a first textile layer (S1) in use on the skin side having a corresponding first opening (A1) to allow the pad in use to contact the skin
a second layer (S2) opposed to the first textile layer with respect to the electrode unit and having a corresponding second opening (A2) to expose a portion of the filiform element at least one electrical terminal (T) fixed in electrical continuity with the filiform element through the second opening and arranged on the surface of the second layer to be in use connected to a control unit configured to receive and process the electrical signal of the pad a mechanical coupling device configured to carry the electronic control unit while it is in electrical continuity with the electrical terminal
at least one layer of adhesive material to attach the first textile layer to the second layer, the electrode unit being interposed, to define a band and to sheath the elongated filiform element with said adhesive
a closure device for releasably connecting a first and a second end portion of the band to each other and allowing in use the band to adhere to the skin of the chest following adjustment, wherein the pad (P) is superimposed on the first textile layer (S1) passing through the first opening (A1), an adhesive layer being present on the pad on an opposite side of the skin in use to attach the pad on the first textile layer.

2. Device according to claim 1, wherein the pad (P) is glued on said first textile layer (S1) out of the first opening (A1) along an elongation direction of filiform element (F).

3. Device according to any of the preceding claims, wherein the electrically conductive polymer is an electrically conductive ink that has been applied by screen-printing.

4. Device according to any of the preceding claims, wherein a first thickness of the ink on the pad (P) is not thicker than a second thickness of the ink on filiform element (F).

5. Device according to any one of the preceding claims, wherein said sheet is a fabric.

6. Device according to any one of the preceding claims, comprising a boss (B), preferably of a metallic material, fixed to the first and / or second layer and having a first surface in use in contact with the skin and a second surface uncovered on the second layer in order to transmit the body heat of a user in use on the second surface.

7. Device according to claim 6, in which the second opening (A2) and the boss (B) are close together so that the electronic control unit, when fixed to the mechanical coupling device, is connected electrically and in heat exchange respectively with the terminal electric and with the stud.

8. Device according to any one of the preceding claims, in which the electrical terminal (T) is in use separated from the skin by means of the first textile layer.

9. Device according to any of the preceding claims, wherein the electric terminals (T) are configured to define also said mechanical coupling device.

10. Device according to claim 9, wherein the electric terminals (T) and, where present, the boss (B) define a non-axisymmetrical shape to define a single angular position to attach said electronic control unit.

11. Device according to any of claims 7 to 9 combined with at least one of claims 9 or 10, wherein at least one of the terminal (T) and the boss (B) is made of a magnetic material.

12. Device according to any one of the preceding claims, in which the sheet is added with ceramic particles to increase the electrical insulation of the applied ink.

13. Top comprising a device according to any one of the preceding claims.

14. Method of manufacturing a device according to claim 3, , comprising the steps of:
Applying the ink on the sheet by screen-printing the ink in an area larger that that of the electrode unit; And
cutting the electrode assembly and the ink from the area.

15. Method according to claim 14, comprising the step of applying a bi-adhesive layer on the sheet on the opposite side of the screen printing before the step of cutting.

## Patentansprüche

1. Textilbandvorrichtung (D), umfassend:
mindestens eine Elektrodeneinheit (E), hergestellt durch Aufbringen eines elektrisch leitfähigen Polymers auf ein flexibles Blatt;
die Elektrodeneinheit umfasst ein Pad (P), das im Gebrauch mit der Haut des Benutzers in Kontakt steht, und ein längliches fadenförmiges Element (F), das eine elektrische Kontinuität mit dem Pad definiert, wobei die Elektrodeneinheit ist aus einem Blatt geschnitten, auf das zuvor das elektrisch leitfähige Polymer aufgebracht wurde;
eine erste Textilschicht (S1), die auf der Hautseite verwendet wird und eine entsprechende erste Öffnung (Al) aufweist, um den Hautkontakt des Pads zu ermöglichen;
eine zweite Schicht (S2), die der ersten Textilschicht in Bezug auf die Elektrodeneinheit gegenüberliegt und eine entsprechende zweite Öffnung (A2) aufweist, um einen Teil des fadenförmigen Elements freizulegen;
mindestens einen elektrischen Anschluss (T), der durch die zweite Öffnung elektrisch mit dem fadenförmigen Element in Kontinuität befestigt ist und auf der Oberfläche der zweiten Schicht angeordnet ist, um mit einer Steuereinheit im Gebrauch verbunden zu werden, die derart konfiguriert ist, um das elektrische Signal des Pads zu empfangen und verarbeiten;
eine mechanische Kopplungsvorrichtung, die die konfiguriert ist, um die elektronische Steuereinheit zu tragen, solange diese elektrisch mit dem elektrischen Anschluss in elektrischen Kontinuität ist;
mindestens eine Schicht Klebstoff zur Befestigung der ersten Textilschicht an der zweiten Schicht, wobei die Elektrodeneinheit dazwischenliegend ein Band definiert und umhüllt das längliche fadenförmige Element mit dem Klebstoff
eine Verschlussvorrichtung, um lösbar ein erstes und ein zweites Endstück des Bandes miteinander zu verbinden und zu gestatten, dass im Gebrauch das Band auf der Haut der Brust haftet, nachdem es geregelt wurde,
wobei das Pad (P) auf der ersten Textilschicht (S1) aufliegt, die durch die erste Öffnung (Al) verläuft, wobei sich auf der dem zu verwendenden Haut gegenüberliegenden Seite des Pads eine Klebeschicht befindet, um das Pad auf der ersten Textilschicht zu befestigen.

2. Vorrichtung nach Anspruch 1, wobei das Pad (P) auf die erste Textilschicht (S1) aus der ersten Öffnung (Al) entlang der Längsrichtung des fadenförmigen Elements (F) geklebt ist.

3. Vorrichtung nach einem der vorhergehenden Ansprüche, wobei das elektrisch fähige Polymer eine elektrisch fähige Tinte ist, die durch Siebdruck aufgebracht wurde.

4. Vorrichtung nach einem der vorhergehenden Ansprüche, wobei eine erste Dicke der Tinte auf dem Pad (P) nicht dicker ist als eine zweite Dicke der Tinte auf dem fadenförmigen Element (F).

5. Vorrichtung nach einem der vorhergehenden Ansprüche, wobei es sich bei dem Blatt um ein Gewebe handelt.

6. Vorrichtung nach einem der vorhergehenden Ansprüche, umfassend einen Ansatz (B), vorzugsweise aus einem metallischen Werkstoff, der an der ersten und/oder zweiten Schicht befestigt ist und eine erste, mit der Haut in Kontakt stehende Oberfläche aufweist, sowie eine zweite, auf der zweiten Schicht unbedeckte Oberfläche, um die Körperwärme eines Benutzers während der Benutzung auf die zweite Oberfläche zu übertragen.

7. Vorrichtung nach Anspruch 6, bei der die zweite Öffnung (A2) und der Ansatz (B) so nahe beieinander liegen, dass die elektronische Steuereinheit, wenn sie an der mechanischen Kupplungsvorrichtung befestigt ist, elektrisch und im Wärmeaustausch mit dem elektrischen Anschluss bzw. mit dem Bolzen verbunden ist.

8. Vorrichtung nach einem der vorhergehenden Ansprüche, bei der der elektrische Anschluss (T) mittels der ersten Textilschicht im Gebrauch von der Haut getrennt ist.

9. Vorrichtung nach einem der vorhergehenden Ansprüche, wobei die elektrischen Anschlüsse (T) so konfiguriert sind, dass sie auch die mechanische Kopplungsvorrichtung definieren.

10. Vorrichtung nach Anspruch 9, wobei die elektrischen Anschlüsse (T) und, falls vorhanden, der Ansatz (B) eine nicht-achsensymmetrische Form aufweisen, um eine einzige Winkelposition zur Befestigung der elektronischen Steuereinheit zu definieren.

11. Vorrichtung nach einem der Ansprüche 7 bis 9 in Verbindung mit mindestens einem der Ansprüche 9 oder 10, wobei mindestens einer der Anschlüsse (T) und der Ansatz (B) aus einem magnetischen Material bestehen.

12. Vorrichtung nach einem der vorhergehenden Ansprüche, wobei der Folie Keramikpartikel zugesetzt werden, um die elektrische Isolation der aufgetragenen Tinte zu erhöhen.

13. Oberteil umfassend eine Vorrichtung gemäß einem der vorhergehenden Ansprüche.

14. Verfahren zur Herstellung einer Vorrichtung nach Anspruch 3, umfassend die folgenden Schritte:
die Tinte wird durch Siebdruck auf ein Blatt in einem Bereich aufgetragen, der größer ist als der der Elektrodeneinheit, und
die Elektrodenanordnung und die Tinte werden aus dem Bereich herausgeschnitten.

15. Verfahren nach Anspruch 14, umfassend den Schritt des Aufbringens einer doppelseitigen Klebeschicht auf das Blatt auf der dem Siebdruck gegenüberliegenden Seite vor dem Schneideschritt.

## Revendications

1. Dispositif textile à bande (D) comprenant :
au moins une unité d'électrode (E) réalisée par application d'un polymère électriquement conducteur sur une feuille flexible ;
l'unité d'électrode comprenant un tampon (P) en contact avec la peau d'un utilisateur en utilisation et un élément filiforme allongé (F), définissant une continuité électrique avec le tampon ;
l'unité d'électrode étant découpée dans une feuille sur laquelle le polymère électriquement conducteur est préalablement appliqué
une première couche textile (S1) utilisée côté peau, présentant une première ouverture correspondante (A1) pour permettre un contact du tampon en utilisation avec la peau ;
une deuxième couche (S2) opposée à la première couche textile par rapport à l'unité d'électrode et présentant une deuxième ouverture correspondante (A2) pour exposer une partie de l'élément filiforme
au moins une borne électrique (T) fixée en continuité électrique avec l'élément filiforme par la deuxième ouverture et disposée sur la surface de la deuxième couche à utiliser reliée à une unité de commande configurée pour recevoir et traiter le signal électrique du tampon
un dispositif de couplage mécanique configuré pour supporter l'unité de commande électronique tout en étant en continuité électrique avec la borne électrique
au moins une couche de matériau adhésif pour fixer la première couche textile à la deuxième couche, l'unité d'électrodes étant interposée pour définir une bande et
recouvrir l'élément filiforme allongé avec ledit adhésif
un dispositif de fermeture pour relier de manière amovible les unes aux autres une première et une deuxième partie d'extrémité de la bande et pour permettre en utilisation l'adhérence de la bande à la peau du thorax suite à un réglage, dans lequel le tampon (P) est superposé à la première couche textile (S1) passant par la première ouverture (Al), une couche adhésive étant présente sur le tampon d'un côté opposé de la peau en utilisation pour fixer le tampon sur la première couche textile.

2. Dispositif selon la revendication 1, dans lequel le tampon (P) est collé sur ladite première couche textile (S1) hors de la première ouverture (Al) le long d'une direction d'allongement de l'élément filiforme (F).

3. Dispositif selon l'une quelconque des revendications précédentes, dans lequel le polymère électriquement conducteur est une encre électriquement conductrice qui a été appliquée par sérigraphie.

4. Dispositif selon l'une quelconque des revendications précédentes, dans lequel une première épaisseur de l'encre sur le tampon (P) n'est pas plus épaisse qu'une deuxième épaisseur de l'encre sur l'élément filiforme (F).

5. Dispositif selon l'une quelconque des revendications précédentes, dans lequel ladite feuille est un tissu.

6. Dispositif selon l'une quelconque des revendications précédentes, comprenant un bossage (B), de préférence en matériau métallique, fixé à la première et/ou deuxième couche et ayant une première surface utilisée en contact avec la peau et une deuxième surface non recouverte sur la deuxième couche afin de transmettre la chaleur corporelle d'un utilisateur en utilisation sur la deuxième surface.

7. Dispositif selon la revendication 6, dans lequel la deuxième ouverture (A2) et le bossage (B) sont proches l'un de l'autre de sorte que l'unité de commande électronique, lorsqu'elle est fixée au dispositif de couplage mécanique, est connectée électriquement et en échange thermique respectivement avec la borne électrique et avec le goujon.

8. Dispositif selon l'une quelconque des revendications précédentes, dans lequel la borne électrique (T) est utilisée séparée de la peau au moyen de la première couche textile.

9. Dispositif selon l'une quelconque des revendications précédentes, dans lequel les bornes électriques (T) sont configurées pour définir également ledit dispositif de couplage mécanique.

10. Dispositif selon la revendication 9, dans lequel les bornes électriques (T) et, lorsqu'il est présent, le bossage (B) définissent une forme non axisymétrique pour définir une position angulaire unique pour le fixage à ladite unité de commande électronique.

11. Dispositif selon l'une quelconque des revendications 7 à 9 combinées avec au moins une des revendications 9 ou 10, dans lequel au moins l'une des bornes (T) et le bossage (B) est constitué d'un matériau magnétique.

12. Dispositif selon l'une quelconque des revendications précédentes, dans lequel la feuille est additionnée de particules de céramique pour augmenter l'isolation électrique de l'encre appliquée.

13. Partie supérieure comprenant un dispositif selon l'une quelconque des revendications précédentes.

14. Procédé de fabrication d'un dispositif selon la revendication 3, comprenant les étapes suivantes :
appliquer l'encre sur la feuille par sérigraphie de l'encre dans une zone plus grande que celle de l'unité d'électrode ; et
découper l'ensemble d'électrode et l'encre de la zone.

15. Procédé selon la revendication 14, comprenant l'étape d'application d'une couche bi-adhésive sur la feuille du côté opposé à la sérigraphie avant l'étape de découpe.
